Europäisches Patentamt

European Patent Office

Office européen des brevets

(11) **EP 1 258 732 A1**

(19)

(12) **EUROPEAN PATENT APPLICATION**
published in accordance with Art. 158(3) EPC

(43) Date of publication:
**20.11.2002 Bulletin 2002/47**

(51) Int Cl.⁷: **G01N 33/70**

(21) Application number: **00900856.6**

(22) Date of filing: **21.01.2000**

(86) International application number:
**PCT/JP00/00276**

(87) International publication number:
**WO 01/053838 (26.07.2001 Gazette 2001/30)**

(84) Designated Contracting States:
**AT BE CH CY DE DK ES FI FR GB GR IE IT LI LU MC NL PT SE**

(71) Applicant: **SEKISUI CHEMICAL CO., LTD.
Osaka-shi, Osaka 530-8565 (JP)**

(72) Inventors:
• **YOKOI, Masayuki
Kusatsu-shi, Shiga 525-0031 (JP)**

• **OKANO, Tsuyoshi
Ibaraki-shi, Osaka 567-0861 (JP)**

(74) Representative: **Crump, Julian Richard John et al
FJ Cleveland,
40-43 Chancery Lane
London WC2A 1JQ (GB)**

(54) **METHOD FOR QUANTITATING CREATININE DISCHARGED IN URINE, METHOD FOR MEASURING URINE COMPONENT AND APPARATUS THEREFOR**

(57)    A method for conveniently quantitating creatinine discharged in urine whereby the total amount of a urine discharged and urine volume in a definite period, which are usable as indicators for finding disease and health care, can be accurately determined without requiring the accumulation of urine; a method for measuring a urine component by using the creatinine level thus measured; and an apparatus for a measuring a urine component.

The first aspect of the invention relates to a method for quantitating creatinine discharged in urine

which comprises the step of determining a regression equation showing the correlation between body weight index and urine creatinine level, and

the step of measuring the body weight index of a subject and substituting it into the above equation. The second aspect of the invention relates to a method for measuring urine components

which comprises the using a value

which is determined by dividing the urine creatinine level determined by the method according to the first aspect of the invention with the creatinine concentration in the urine specimen and multiplying the quotient by the concentration of the substance to be determined in the urine specimen.

The third aspect of the invention relates to an apparatus for measuring urine components which comprises using the method according to the second aspect of the invention.

**Description**

TECHNICAL FIELD

The present invention relates to a method for quantitating creatinine discharged in urine, method for measuring urine component and an apparatus therefor.

BACKGROUND ART

The measurement of components in urine such us urine glucose, urine protein and urine salt is a time-honored practice for the discovery of diseases or for use as benchmarks in health care guidance. By way of illustration, a high level of urine glucose suggests diabetes, a high level of urine protein indicates the likelihood of nephritis, and a high level of urine salt suggests hypertension. Since detection of such a benchmark sign justifies a closer examination to be made and the proper health care guidance or therapy of the illness to be instituted, determination of the urine composition is of considerable use in the field of medical care.

In the determination of the urine composition, the amount and concentration of creatinine discharged in urine are also measured. Since creatinine occurs in the human urine without exception, it has been utilized in correcting the urine concentration in the assay of other urine components. Moreover, as concentration of the creatinine discharged in urine sometimes suggests the likelihood of a disease of the kidney or uremia, measurement of the creatinine discharged in urine is generally considered important.

Various techniques are available for the determination of urine components; for example, for the determination of urine salt which is one of urine components, the method comprising the use of a test paper for sodium chloride in urine and the method comprising the use of the measuring instrument disclosed in Japanese Kokai Publication Hei-08-75727, among others, have been practiced. However, the urine is influenced by the subject's exercise amount, diets, drinking water and other factors and the concentrations of its components vary from one voidance to another. Therefore, these methods are incapable of determining the respective excretions in the urine over one day or a few days, although the concentrations of urine components in any one batch of urine or the total amounts of the respective components in each voided urine specimen can be measured. Moreover, since the urine concentration and volume vary from one voidance of urine to another, measurement data tend to vary and, hence, inadequate for use as benchmarks in health care guidance, for instance.

Therefore, in order to perform high-precision determinations by any of the above methods, it is necessary to accumulate urine for at least one day (hereinafter referred to as accumulation of urine) but for the accumulating of urine for one day, it is not only necessary to provide a utensil such as a urine accumulation bag but also requires much labor, and, in addition, the burden on the subject is remarkable. Furthermore, the urine must be accumulated for as long as 24 hours and this causes another problem that urine components undergo change due to aging in the course.

SUMMARY OF THE INVENTION

In the above state of the art, the present invention has for its object to provide a method for conveniently quantitating creatinine discharged in urine whereby the total amount of a urine discharged and urine volume in a definite period, which are usable as indicators for finding disease and health care, can be accurately determined without requiring accumulation of urine; a method for measuring a urine component by using the creatinine level thus measured; and an apparatus for a measuring a urine component.

The first aspect of the invention relates to a method for quantitating creatinine discharged in urine

which comprises the step of determining a regression equation showing the correlation between body weight index and creatinine level discharged in urine, and

the step of measuring the body weight index of a subject and substituting it into the said regression equation.

The second aspect of the invention relates to a method for measuring urine components practiced with the use of a value which is determined by dividing the urine creatinine level determined by the method according to the first aspect of the invention with the creatinine concentration in the urine specimen and multiplying the quotient by the concentration of the substance to be determined in the urine specimen.

The third aspect of the invention relates to an apparatus for measuring urine components with the use of the method according to the second aspect of the invention.

DISCLOSURE OF THE INVENTION

The present invention is now described in detail.

In the method for quantitating creatinine discharged in urine according to the first aspect of the invention, a regression equation showing the correlation between body weight index and creatinine level discharged in urine in a definite period is determined in the first place. The "definite period" referred to above may be any duration of time that is not less than one day and, thus, may for example be one day, 2 days, or 2.5 days. However, the upper limit is preferably not more than 7 days. As used in this specification, the term "body weight index" is a value representing the body mass related with the voidance of urine. As said body weight index, there can be used body weight, lean body mass, body fat mass, BMI, or the like.

As used in this specification, said lean body mass means the body mass after subtraction of body fat mass from total body weight [(body weight) - (body fat mass)], the body weight after subtraction of body fat mass and bone mass from total body weight [(body weight) - (body fat mass)- (bone mass)], or the total protein mass of a human body.

The method of determining said lean body mass is not particularly restricted but includes, among others, indirect methods such as the method which comprises measuring whole-body density by, for example, the water displacement technique and calculating lean body mass and the method which comprises carrying out a calculation based on the sebum thickness; direct methods for determining lean body mass or total protein mass, such as the DEXA method, the method using the radioactive substance $^{40}$K ($K^{40}$ method) and the method using near-infrared rays; and the method involving a human body impedance measurement. Among these, the method involving a human body impedance measurement is particularly preferred.

In the above indirect methods, body fat mass must be measured. The body fat mass can be measured with a commercial body fat meter. With an apparatus allowing a determination of body fat percentage, the body fat mass can be calculated from body weight and body fat percentage.

The apparatus for use in measuring said human body impedance to determine lean body mass is not particularly restricted, whether in the mounting sites of measuring electrodes or in the measuring frequency to be used, provided that it is capable of measuring body fat mass or lean body mass. As such apparatus, commercial instruments can be utilized. For example, the body fat meter-equipped body weight scale TBF534 (manufactured by Tanita), Omron body fat meter HBF301 (manufactured by Omron), the multi-frequency body fat meter MLT100 (manufactured by Sekisui Chemical), and Fat Percenter S-201 (manufactured by Sekisui Chemical) can be used with advantage. Particularly preferred is Fat Percenter S-201. The Fat Percenter S-201 mentioned above is designed to apply high-frequency (50 kHz) waves to the human body and measure the impedance to find body fat mass, lean body mass, and body water content.

In the first aspect of the invention, not only the above methods but the method which comprises using the body mass index (BMI) to calculate said lean body mass can be employed. Incidentally, the BMI referred to above is the value expressed by the following equation;

$$BMI = (body\ weight)/(body\ height)^2$$

where the unit of body weight is kg and the unit of body height is m. Thus, for example, the BMI of a human individual with a body weight of 60 kg and a body height of 1.7 m is 21 [$kg \cdot m^{-2}$].

Because said BMI is correlated with body fat percentage, the body fat percentage can be calculated by means of a regression equation correlating BMI with body fat percentage. The regression equation mentioned above is not particularly restricted but may for example be a linear, quadratic, or logarithmic regression equation. In the present invention, any of such regression equations can be used insofar as the correlation coefficient between said BMI and body fat percentage that can be obtained is not less than 0.6, preferably not less than 0.8.

When a linear regression equation is used as said regression equation, body fat percentage (%) can be expressed by the following equation (1).

$$(Body\ fat\ percentage) = a\ x\ (BMI) + b \qquad (1)$$

where a is -2 to 2 and b is -10 to 10.

By way of illustration, in the case where a = 0.5 and b = 10 in the above equation (1), the body fat percentage of the above-mentioned individual having a body weight of 60 kg and a body height of 1.7 m is calculated to be 20.5%.

Lean body mass (kg) can be calculated by substituting the body fat percentage value calculated by means of the above regression equation (1) into the following equation (2).

$$(Lean\ body\ mass) = [(100 - (body\ fat\ percentage)\}/100]\ x\ (body\ weight) \qquad (2)$$

Thus, the lean body mass of a human individual having a body weight of 60 kg and a body fat percentage of 20.5% is 48 kg.

In the first aspect of the invention, a regression equation showing the correlation between the above-mentioned body weight index and creatinine level discharged in urine for definite period is determined. As said body weight index and said creatinine level discharged in urine for definite period, the respective data so far generated and accumulated are used. The above regression equation is not particularly restricted but may for example be a linear, quadratic or logarithmic regression equation. In the first aspect of the invention, any of such regression equations is acceptable only provided that the correlation coefficient between the above-mentioned body weight index and creatinine level discharged in urine for definite period is not less than 0.6, preferably not less than 0.8.

In the case where a linear regression equation is used as said regression equation and said definite period is one day, the relation between lean body mass and one-day creatinine level discharged in urine can be expressed by the following equation (3). Moreover, the relation between body weight and one-day creatinine level discharged in urine can be expressed by the following equation (4).

$$\text{(One-day creatinine level discharged in urine/mg)} = c \times \text{(lean body mass/kg)} + d \tag{3}$$

$$\text{(One-day creatinine level discharged in urine/mg)} = e \times \text{(body weight/kg)} + f \tag{4}$$

wherein c is 5 to 45, d is 0 to 5, e is 5 to 35, and f is 0 to 5.

By way of illustration, in the case where c = 5 and d = 3 in the above equation (3), the one-day creatinine level discharged in urine of a human individual with a lean body mass of 48 kg is 243 mg.

For calculating said lean body mass by utilizing said BMI, the relation between BMI and one-day creatinine level discharged in urine can be reduced to the following equation (5).

$$\text{(One-day creatinine level discharged in urine/mg)} = c \times [[100 - \{a$$

$$\times (BMI/kg \cdot m^{-2}) + b\}]/100] \times \text{(body weight/kg)} + d \tag{5}$$

where a, b, c, and d are as defined above.

The variables a, b, c, d, e, and f in the above regression equations (3) to (5) are classified according to sex, age and other factors and set appropriately.

In the first aspect of the invention, by measuring the body weight index of a subject and substituting it into the above regression equation, the creatinine level discharged in urine for definite period is calculated. The body weight index of said subject can be freely selected according to the body weight index used in the regression equation to be utilized.

Furthermore, in the first aspect of the invention, by using the creatinine level discharged in urine for definite period as calculated from the above regression equation and the measured creatinine level discharged in urine and urine volume of the voided urine specimen from one micturition or the measured creatinine concentration of the urine specimen, the urine volume for definite period can be calculated without actual accumulation of urine.

For example, assuming that said "definite period" is one day, the one-day urine volume can be calculated by means of either the following equation (6) or the following equation (7).

$$\text{(One-day urine volume)} = [\text{(one-day creatinine level discharged}$$

$$\text{in urine)}/\text{(amount of creatinine in test urine)}] \times \text{(volume of test urine)} \tag{6}$$

$$\text{(One-day urine volume)} = \text{(one-day creatinine level discharged}$$

$$\text{in urine)}/\text{(creatinine concentration of test urine)} \tag{7}$$

Furthermore, when the method of estimating creatinine level discharged in urines according to the first aspect of the invention is used, by measuring the total amount or concentration of any given component of interest in the urine specimen from one micturition, not only the discovery of a disease is feasible but also the total urine excretions of other urine components, which may be used as benchmarks for health care guidance, for instance, within definite period can be calculated.

The urine component referred to above is not particularly restricted but includes, among others, urinary glucose, urinary salt, urinary albumin, urinary protein, urinary urobilinogen, urinary bilirubin, urinary N-acetyl-$\beta$-D-glucosaminidase (urine NAG), urinary $\beta$2 microglobulin, urinary $\alpha$1 microglobulin, urinary ketones, urinary polyamine, urinary 5-hydroxyindoleacetic acid (urine 5-HIAA), GTP, $\gamma$-GTP, GOP, amylase, cholesterol, lipids, and calcium.

Assuming that said "definite period" is one day, the one-day total urinary excretion of each of said urine components can be calculated by means of either the following equation (8) or the following equation (9).

$$\text{(One-day total urinary excretion of a component of interest)}$$

$$= [\text{(one-day creatinine level discharged in urine)/(total}$$

$$\text{creatinine in test urine)]} \times \text{(total amount of the component of interest in test urine)} \qquad (8)$$

$$\text{(One-day total urinary excretion of a component of interest)}$$

$$= [\text{(one-day creatinine level discharged in urine) / (creatinine}$$

$$\text{concentration of test urine)]} \times \text{(concentration of the component of interest in test urine)} \qquad (9)$$

Since, in the method for quantitating creatinine discharged in urine according to the first aspect of the invention, a regression equation for correlation is derived from a body weight index and the creatinine level discharged in urine for definite period, the creatinine level discharged in urine during any definite period can be easily and conveniently estimated by measuring the body weight index.

Furthermore, since the creatinine level discharged in urine estimated by the method of estimating creatinine level discharged in urine according to the first aspect of the invention is the creatinine level discharged in urine for definite period, it can be utilized for estimating accurate values of urine volume and urinary excretions of various components during definite period from one urine batch even if the concentration of urine varies from one voidance to another, thus dispensing with accumulation of urine.

The second aspect of the present invention is directed to a method for measuring urine components practiced with the use of a value which is determined by dividing the urine creatinine level determined by the method according to the first aspect of the invention with the creatinine concentration in the urine specimen and multiplying the quotient by the concentration of the substance to be determined in the urine specimen. Therefore, by utilizing this method of the second aspect of the invention, the total cumulative amount of each urine component, for instance, can be easily and conveniently determined, thus lessening the burden on the subject.

The third aspect of the invention is concerned with an apparatus for measuring urine components with the use of the method of the second aspect of the invention.

BEST MODE FOR CARRYING OUT THE INVENTION

The following examples illustrate the present invention in further detail without defining the scope of the invention. In these examples, the "definite period" is invariably "one day".

Example 1

Determination by the lean body mass method

For 5 men and 5 women in their twenties, 5 men and 5 women in their forties, and 5 men and 5 women in their sixties, the determination of lean body mass was carried out using Fat Percenter S-201 (an body fat meter manufactured by Sekisui Chemical). Then, using fx-7200G (a programmable electronic calculator manufactured by Casio), the one-day creatinine level discharged in urine was calculated in accordance with the following equation.

(Male)

Aged 20 to 39 years: (one-day creatinine level discharged in

urine/mg) = 26 x (lean body mass/kg) + 2

Aged 40 to 59 years: (one-day creatinine level discharged in

urine/mg) = 25 x (lean body mass/kg) + 2

Aged 60 and older: (one-day creatinine level discharged in

urine/mg) = 20 x (lean body mass/kg) + 2

(Female)

Aged 20 to 39 years: (one-day creatinine level discharged in

urine/mg) = 25 x (lean body mass/kg) + 1

Aged 40 to 59 years: (one-day creatinine level discharged in urine/mg) = 21 x (lean body mass/kg) + 1

Aged 60 and older: (one-day creatinine level discharged in

urine/mg) = 18 x (lean body mass/kg) + 1

In the above population, the correlation between the total creatinine level discharged in urine found by one-day-long accumulation of urine and the one-day creatinine level discharged in urine estimated by the lean body mass method was found to be r = 0.98; indicating that a good correlation can be obtained.

Example 2

The one-day creatinine level discharged in urine determined in Example 1 was divided by the amount of creatinine in a voided urine specimen at the time of determination and the quotient was multiplied by the urine volume at said time of determination to calculate the one-day urine volume. The correlation between this value and the value found by actual one-day accumulation of urine was found to be r = 0.92, indicating that a good correlation can be obtained.

Example 3

The one-day creatinine level discharged in urine determined in Example 1 was divided by the creatinine concentration of a voided urine specimen at the time of determination and the quotient was multiplied by the urine glucose concentration at said time of determination to calculate the one-day urine glucose value. The correlation between this value and the value found by actual one-day accumulation of urine was found to be r = 0.91.

Example 4

The one-day creatinine level discharged in urine determined in Example 1 was divided by the creatinine concentration of a voided urine specimen at the time of determination and the quotient was multiplied by the concentration of each of the components indicated in Table 1 to calculate the one-day excretion of the particular component. The correlation between this value and the value found by actual accumulation of urine was determined. The results are presented in Table 1.

Table 1

| Component of interest | Correlation with one-day urine volume |
|---|---|
| Sodium chloride | 0.98 |
| Amylase | 0.97 |
| GTP | 0.94 |
| γ-GTP | 0.95 |
| Cholesterol | 0.93 |
| Calcium | 0.97 |

Example 5

Determination by the body weight method

For 5 men and 5 women in their twenties, 5 men and 5 women in their forties, and 5 men and 5 women in their sixties, body weight was taken using a body weight scale. Then, using fx-7200G (a programmable electronic calculator manufactured by Casio), the one-day creatinine level discharged in urine was calculated by means of the following equation.

(Male)

Aged 20 to 39 years: (one-day creatinine level discharged in urine/mg) = 22 x (body weight/kg) + 1

Aged 40 to 59 years: (one-day creatinine level discharged in urine/mg) = 21 x (body weight/kg) + 1

Aged 60 and older: (one-day creatinine level discharged in urine/mg) = 17 x (body weight/kg) + 1

(Female)

Aged 20 to 39 years: (one-day creatinine level discharged in urine/mg) = 19 x (body weight/kg) + 1

Aged 40 to 59 years: (one-day creatinine level discharged in urine/mg) = 17 x (body weight/kg) + 1

Aged 60 and older: (one-day creatinine level discharged in urine/mg) = 14 x (body weight/kg) + 1

In the above population, the correlation between the total creatinine excretion found by one-day accumulation of urine and the one-day creatinine level discharged in urine determined by the body weight method was found to be r = 0.84, indicating that a good correlation can be obtained.

Example 6

The one-day creatinine level discharged in urine determined in Example 5 was divided by the creatinine value of a voided urine specimen at the time of determination and the quotient was multiplied by the urine volume at the time of determination to calculate the one-day urine volume. The correlation between this value and the value found by actual one-day accumulation of urine was r = 0.82.

Example 7

The one-day creatinine level discharged in urine determined in Example 5 was divided by the creatinine concentration of a voided urine specimen at the time of determination and the quotient was multiplied by the concentration of urine glucose at the time of determination to calculate the one-day urine glucose value. The correlation between this value and the value found by actual one-day actual accumulation of urine was r = 0.81.

Example 8

The one-day creatinine level discharged in urine determined in Example 5 was divided by the creatinine concentration of a voided urine specimen at the time of determination and the quotient was multiplied by the concentration of each of the components indicated in Table 2 at the time of determination to calculate the corresponding one-day value. The correlation between this value and the value found by actual accumulation of urine was calculated. The results are presented in Table 2.

Table 2

| Component of interest | Correlation with one-day urine volume |
|---|---|
| Sodium chloride | 0.88 |
| Amylase | 0.87 |
| GTP | 0.84 |
| γ-GTP | 0.85 |
| Cholesterol | 0.83 |
| Calcium | 0.87 |

Example 9

Determination by the body fat mass method

For 5 men and 5 women in their twenties, 5 men and 5 women in their forties, and 5 men and 5 women in their sixties, body fat mass was measured with Omron body fat meter HBF301 (a body fat meter manufactured by Omron). Then, using fx-7200G (a programmable electronic calculator manufactured by Casio), the one-day creatinine level discharged in urine was calculated according to the following equation.

(Male)

Aged 20 to 39 years: (one-day creatinine level discharged in

urine/mg) = 26 x [(body weight) - (body fat mass/kg)] + 1

Aged 40 to 59 years: (one-day creatinine level discharged in

urine/mg) = 25 x [(body weight) - (body fat mass/kg)] + 1

Aged 60 and older: (one-day creatinine level discharged in

urine/mg) = 20 x [(body weight) - (body fat mass/kg)] + 1

(Female)

Aged 20 to 39 years: (one-day creatinine level discharged in

urine/mg) = 25 x [(body weight) - (body fat mass/kg)] + 1

Aged 40 to 59 years: (one-day creatinine level discharged in urine/mg) = 21 x [(body weight) - (body fat mass/kg)] + 1

Aged 60 and older: (one-day creatinine level discharged in

urine/mg) = 18 x [(body weight) - (body fat mass/kg)] + 1

In the above population, the correlation between the total creatinine value found by one-day accumulation of urine and the one-day creatinine level discharged in urine determined by the body fat mass method was found to be r = 0.84, indicating that a good correlation can be obtained.

Example 10

The one-day creatinine level discharged in urine determined in Example 9 was divided by the amount of creatinine in a voided urine specimen at the time of determination and the quotient was multiplied by the urine volume at the time of determination to calculate the one-day urine volume. The correlation between this value and the value found by one-day accumulation of urine was r = 0.82.

Example 11

The one-day creatinine level discharged in urine determined in Example 9 was divided by the creatinine concentration of a voided urine specimen at the time of determination and the quotient was multiplied by the concentration of urine glucose at the time of determination to calculate the one-day urine volume. The correlation between this value and the value found by one-day accumulation of urine was r = 0.81.

Example 12

The one-day creatinine level discharged in urine determined in Example 9 was divided by the creatinine concentration of a voided urine specimen at the time of determination and the quotient was multiplied by the concentration of each of the components indicated in Table 3 at the time of determination to find the corresponding one-day value. The correlation of each of these values and the corresponding value found by actual accumulation of urine is presented in Table 3.

Table 3

| Component of interest | Correlation with one-day urine volume |
|---|---|
| Sodium chloride | 0.94 |
| Amylase | 0.93 |
| GTP | 0.91 |
| γ-GTP | 0.89 |
| Cholesterol | 0.86 |
| Calcium | 0.87 |

Example 13

Determination by the BMI method

For 5 men and 5 women in their twenties, 5 men and 5 women in their forties, and 5 men and 5 women in their sixties, the body height and body weight were taken using a body height measure and a flat bathroom scale, respectively. The data were substituted into the equation BMI = (body weight) / (body height)$^2$ to calculate the BMI. Then, using fx-7200G (a programmable electronic calculator manufactured by Casio), the one-day creatinine level discharged in urine was calculated by means of the following equation.

(Male)

Aged 20 to 39 years: (one-day creatinine level discharged in urine/mg) = 22 x

(body weight/kg) x [{100-(BMI/kg·m$^{-2}$ + 2)}/100]

Aged 40 to 59 years: (one-day creatinine level discharged in urine/mg) = 21 x

(body weight/kg) x [{100-(BMI/kg·m$^{-2}$ + 2)}/100]

Aged 60 and older: (one-day creatinine level discharged in urine/mg) = 17 x (body

weight/kg) x [{100-(BMI/kg·m$^{-2}$ + 2)}/100]

(Female)

Aged 20 to 39 years: (one-day creatinine level discharged in urine/mg) = 19 x

(body weight/kg) x [{100-(BMI/kg·m$^{-2}$ + 4)}/100]

Aged 40 to 59 years: (one-day creatinine level discharged in urine/mg) = 17 x

(body weight/kg) x [{100-(BMI/kg·m$^{-2}$ + 4)}/100]

Aged 60 and older: (one-day creatinine level discharged in urine/mg) = 14 x (body

weight/kg) x [{100-(BMI/kg·m$^{-2}$ + 4)}/100]

In the above population, the correlation between the total creatinine value found by one-day accumulation of urine and the one-day creatinine level discharged in urine determined by the BMI method was r = 0.74, indicating that a good correlation can be obtained.

Example 14

The one-day creatinine level discharged in urine calculated in Example 13 was divided by the creatinine concentration of a voided urine specimen at the time of determination and the quotient was multiplied by the urine volume at the time of determination to calculate the one-day urine volume. The correlation between this value and the value found by actual one-day accumulation of urine was r = 0.72.

Example 15

The one-day creatinine level discharged in urine calculated in Example 13 was divided by the creatinine concentration of a voided urine specimen at the time of determination and the quotient was multiplied by the concentration of urine glucose at the time of determination to calculate the one-day urine glucose value. The correlation between this value and the value found by the actual one-day accumulation of urine was r = 0.71.

Example 16

The one-day creatinine level discharged in urine calculated in Example 13 was divided by the creatinine concentration of a voided urine specimen at the time of determination and the quotient was multiplied by the concentration of each of the components indicated in Table 4 at the time of determination to calculate the corresponding one-day value. The correlation between each of these values and the corresponding value found by actual accumulation of urine was investigated. The results are presented in Table 4.

Table 4

| Component of interest | Correlation with one-day urine volume |
|---|---|
| Sodium chloride | 0.74 |
| Amylase | 0.73 |
| GTP | 0.71 |
| γ-GTP | 0.79 |
| Cholesterol | 0.76 |
| Calcium | 0.77 |

INDUSTRIAL APPLICABILITY

By the measurement methods according to the first and second aspect of the inventions which are constituted as above, the creatinine level discharged in urine for definite period can be easily estimated by measuring the body weight index and making a computation. Therefore, the exact urine volume for definite period and the exact amounts of urine components for definite period, which can be used as benchmarks for the discovery of diseases and in health care guidance, can be easily estimated from one urine batch without requiring the accumulation of urine.

**Claims**

1.  A method for quantitating creatinine discharged in urine
    which comprises the step of determining a regression equation showing the correlation between body weight index and creatinine level discharged in urine, and
    the step of measuring the body weight index of a subject and substituting it into the said regression equation.

2.  The method for quantitating creatinine discharged in urine according to Claim 1,
    wherein the body weight index is either body weight or lean body mass.

3.  The method for quantitating creatinine discharged in urine according to Claim 2,
    wherein the lean body mass is

    $$[(body\ weight) - (body\ fat\ mass)].$$

4.  The method for quantitating creatinine discharged in urine according to Claim 2,
    in which the lean body mass is calculated by the method which comprises determining a body fat percentage by a method
    which comprises the step of determining a regression
    equation showing the correlation between BMI,
    which is expressed by the equation;

    $$BMI = (body\ weight)/(body\ height)^2,$$

    and body fat percentage and substituting the value of body fat percentage into said regression equation, and substituting the resulting body fat percentage value into the following equation;

    $$Lean\ body\ mass = [\{100 - (body\ fat\ percentage)\}/100] \times (body\ weight).$$

5.  The method for quantitating creatinine discharged in urine according to Claim 2,
    wherein the lean body mass is calculated by using the whole-body density, near-infrared method, DEXA method, $K^{40}$ method, sebum thickness or human body impedance value.

6.  A method for measuring urine components
    which comprises the using a value
    which is determined by dividing the urine creatinine level determined by the method according to any of Claims 1 to 5 with the creatinine concentration in the urine specimen and multiplying the quotient by the concentration of the substance to be determined in the urine specimen.

7.  An apparatus for measuring urine components
    which comprises using the method according to Claim 6.

## INTERNATIONAL SEARCH REPORT

| International application No. |
|---|
| PCT/JP00/00276 |

| A. CLASSIFICATION OF SUBJECT MATTER |
|---|
| Int.Cl⁷ G01N33/70 |

According to International Patent Classification (IPC) or to both national classification and IPC

| B. FIELDS SEARCHED |
|---|

Minimum documentation searched (classification system followed by classification symbols)
Int.Cl⁷ G01N33/70

Documentation searched other than minimum documentation to the extent that such documents are included in the fields searched

| Jitsuyo Shinan Koho | 1922-1996 | Toroku Jitsuyo Shinan Koho | 1994-2000 |
| Kokai Jitsuyo Shinan Koho | 1971-2000 | Jitsuyo Shinan Toroku Koho | 1996-2000 |

Electronic data base consulted during the international search (name of data base and, where practicable, search terms used)

| C. DOCUMENTS CONSIDERED TO BE RELEVANT |
|---|

| Category* | Citation of document, with indication, where appropriate, of the relevant passages | Relevant to claim No. |
|---|---|---|
| Y | Masamitsu KANAI ed.,"Rinshou Kensahou Teiyou, the 30th printing", Kanehara Shuppan, 20 August, 1993 (20.08.93), pp.150~151 | 1~7 |
| Y | Shouji SUZUKI, "Igaku Daijiten, the 16th printing ", Nanzando, 11 February, 1978 (11.02.1978), p.526 | 1~7 |

| ☐ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 12 April, 2000 (12.04.00) | 25 April, 2000 (25.04.00) |

| Name and mailing address of the ISA/ | Authorized officer |
|---|---|
| Japanese Patent Office | |
| Facsimile No. | Telephone No. |

Form PCT/ISA/210 (second sheet) (July 1992)